# EUROPEAN PATENT APPLICATION

(11) **EP 3 542 699 A1**
(43) Date of publication of application: **25.09.2019**
(21) Application number: 19161407.2
(22) Date of filing: 07.03.2019
(51) Int. Cl.: A61B 1/00, A61B 1/06, A61B 1/24

(54) **HANDHELD DEVICE FOR DIAGNOSING AN IN-VIVO TARGET**

(30) Priority: 22.03.2018 CH 3902018
(71) Applicant: Mectron S.p.A., 16042 Carasco GE (IT)
(72) Inventor: Jacocagni, Fabio, 16042 Carasco (IT); Jacocagni, Fulvio, 16042 Carasco (IT); Della Rosa, Jacopo, 16042 Carasco (IT); Leonardi, Riccardo, 16042 Carasco (IT); Giovanacci, Ilaria, 16042 Carasco (IT)
(74) Representative: Spierenburg, Pieter

(57) **Abstract**

An novel intraoral inspection apparatus (1) for diagnosing the physiological and pathological status of an in vivo target tissue (30) is described which comprises a light source unit (8) provided with at least one color-producing light source capable of inducing fluorescent lights from healthy and/or diseased sites by irradiating the target tissue. Further a wavelength adjusting device (12) is provided having filter means that transmits a light having a wavelength of a fluorescent light from a predetermined diseased site and blocks or attenuates a light having a wavelength other than this wavelength. An imaging device (17) receives the fluorescent light transmitted through the filter means, and converts them into electrical signals. The apparatus (1) is a hand-held device comprising an elongated body (2) including a proximal end (3) and a distal end (4), the body (2) being configured to position the distal end (4) proximate to the target tissue (30), whereas the light source unit (8) is provided at the proximate end (3), and an optical beam splitter (11) with interferential filters (13) is provided in front of the light source unit (8) to remove unwanted wavelengths and to collimate the light beam into the elongated body (2).

## Description

The invention is related to a handheld device for diagnosing an in-vivo target tissue device according to the preamble of claim 1.

### FIELD OF THE INVENTION

There is an increased need for early diagnosis of the physiological and pathological status of an in-vivo target tissue such as derma or bone tissues or oral mucosa.

### BACKGROUND OF THE INVENTION

Devices for diagnosing an in-vivo target tissue are well-known, for example from US patent no. 6,110,106 which describes an apparatus that permit the direct viewing of induced tissue fluorescence by a human viewer through an endoscope without the need for bulky and expensive auxiliary apparatus such as imaging devices, sensor arrays, analyzing systems and computer hardware and software. The apparatus provide an excitation energy, such as blue light, that is transmitted to the target, and then the endoscope collects the emitted response, which is typically fluorescent light of a wavelength longer that the UV of blue light used to induce the fluorescence. The endoscope then transmits the response through a series of filters in the endoscope and directly to the eye of a human user.

Since aforementioned device is provided for investigation by a human user, one can only obtain an instant image of a patient's oral mucosa. However, comparing results of previous investigations or with results of healthy oral mucosa is not possible. Moreover the adjustable wavelength ratio scaler is quite bulky and laborious to handle.

In US patent no. 6,325,623 a dental curing device with an expanded functionality for diagnosing abnormalities by observation of native or induced fluorescence in the oral cavity is described. An optical assembly consisting of a short pass or band pass filter and a spacer is applied to the output distal end of the dental light curing device. A long pass filter is applied to the attenuating filter or the spacer is made as a long pass filter. The diagnosing sites can be seen by the human eye through the spacer.

The above mentioned curing device can only give a preliminary indication for a dentist for abnormalities as dysplastic or malignant lesions. However, a profound investigation and comparison with previous results is not possible.

US patent no. 7,365,844 mentions systems and methods for diagnosing epithelial neoplasia and other conditions which includes providing the human eyes with a filter to observe the autofluorescence of a tissue sample. Using optimized wavelengths form a filtered light source, a sample is illuminated. The radiation from the sample is filtered to enhance the contrast between the normal sample and a diseased sample observable by the human eye.

The system described in this document includes a white light source such as a Xenon, Tungsten or Mercury lamp, which directs a light beam to a cold mirror to reject radiation below 300 nm and above 625 nm, preventing UV and infrared radiation from the light source to reach a filter wheel which houses at least one bandpass filter. The wavelengths passing the filter wheel are focused on a light guide, which is used to illuminate a sample, such as an entire oral cavity or a tissue sample. The radiation or fluorescence from the sample is observed at right angles with a viewing device, such as glasses containing a long bandpass filter. The viewing device may be a camera containing the long bandpass filter used to document autofluorescence.

The system is described only in principle setup and has the same disadvantages as in the previous documents.

Further, European patent application no. EP 2 554 107 A1 describes an intraoral inspection apparatus for dental use which is capable of distinguishing and detecting more kinds of diseased sites and the like with one apparatus. The intraoral inspection apparatus comprises a light source unit which is provided with color-producing light sources capable of inducing fluorescent lights from diseased sites by irradiating an oral cavity. The light source is provided with a hood as a cylindrical member with a bottom plate, and a circular hole is formed on the bottom plate. On the bottom plate an inner ring of white light sources (white LEDs), a middle ring of first color-producing light sources (blue LEDs) and an outer ring of second color-producing light sources (red LEDs) are arranged. Further a wavelength adjusting device is provided, which is designed a case with three filters in line: a filter for a white light source, a first filter to be used with a first color-producing light source and a second filter to be used with a second color-producing light source. The wavelength adjusting device is slidably arranged between the light source and an imaging device like CCD or CMOS. Thus, the imaging device receives the fluorescent light transmitted through the first filter and the fluorescent light transmitted to the second filter dependent from the filter position of the wavelength adjusting device, and converts them into electrical signals.

The apparatus described in this document is only a principle setup, which will lead to a quite bulky device.

### OBJECT OF THE INVENTION

It is an object of the present invention to provide an intraoral inspection apparatus for diagnosing the physiological and pathological status of an in vivo target tissue, which is light weigthed and easy to handle in order to be used by one hand, whereas early detection of potential lesions can be obtained and subsequent pictures of the target tissue can be compared which each other.

### SUMMARY OF THE INVENTION

This object is obtained by an intraoral inspection apparatus for diagnosing the physiological and pathological status of an in vivo target tissue being derma or bone tissues or oral mucosa with the features of claim 1. The apparatus comprises a light source unit which is provided with at least one color-producing light source capable of inducing fluorescent lights from healthy and/or diseased sites by irradiating the target tissue. A wavelength adjusting device is provided having filter means that transmits, among the fluorescent lights, a light having a wavelength of a fluorescent light from a predetermined diseased site and blocks or attenuates a light having a wavelength other than this wavelength. Further, an imaging device is provided which receives the fluorescent light transmitted through the filter means, and converts them into electrical signals. The apparatus is a hand-held device comprising an elongated body including a proximal end and a distal end, whereas the body being configured to position the distal end proximate to the target tissue. The light source unit is provided at the proximate end, and an optical beam splitter with interferential filters is provided in front of the light source unit to remove unwanted wavelengths and to collimate the light beam into the elongated body.

In a preferred embodiment of the intraoral inspection apparatus the light source unit is an array of LEDs.

In another embodiment the imaging device is a CCD or CMOS sensor.

In a further embodiment the wavelength adjusting device is a filter wheel with a plurality of filter sections each having an individual bandpass characteristic.

In a further preferred embodiment the wheel is driven by a stepper motor.

In another preferred embodiment a stroboscopic means is related to the light source unit for stroboscopic excitation of the illumination light, and an acquisition means related to the imaging device is synchronising the electrical signals obtained by the imaging device from the fluorescent light with the stroboscopic excitation of the illumination light.

In a further embodiment the first and second optical fibers are covered by a disposable sheath.

Preferrably the disposable sheath comprises a disposable cap. In addition the disposable cap may be fixed at a predetermined angle to the optical axis.

### BRIEF DESCRIPTION OF THE DRAWINGS

The invention will now be described in greater detail, by way of example, with reference to the accompanying drawings, in which:
Fig. 1 shows an intraoral inspection apparatus according to the present invention,
Fig. 2A, 2B, 2C show three versions of a disposable cover with a disposable cap,
Fig. 3 shows the optical beam splitter for a first light source in more detail,
Fig. 4 and 5 show the use of the optical beam splitter for a second and third light source,
Fig. 6 shows the receiving of the auto-fluorescence light by the camera,
Fig. 7 shows the spectra of the transmitted light,
Fig. 8 shows the filter wheel in a front view,
Fig. 9 shows the spectra of the emitted light and the receiving light with the filter bands, respectively, and
Fig. 10 shows the basic principles of the diagnosing method.

### DETAILED DESCRIPTION OF THE PREFERRED EMBODIMENTS

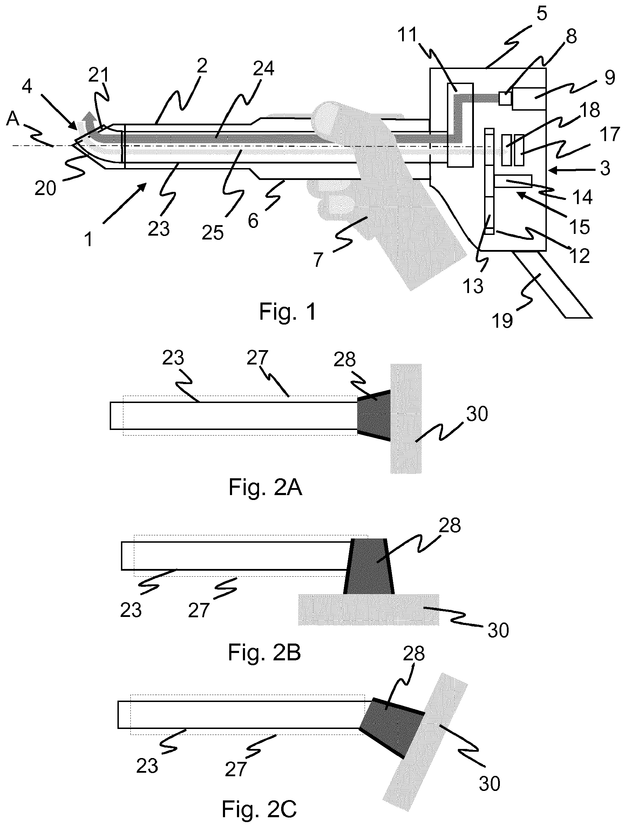

In Fig. 1 an intraoral inspection apparatus 1 as preferred embodiment of the invention with an elongated body 2 with a proximal end 3 and a distal end 4 is depicted. The apparatus 1 is a hand-held device or handpiece with a rear part 5 and a graspable middle part 6 which can be grasped by a user's hand 7. In the rear part 5 there is provided a light source unit 8 optionally with a cooling system 9 which has an aluminum heat sink, a fan and openings. In front of the light source 8 an optical beam splitter 11 is provided. Further in the rear part 5 a wave adjusting device 12 designed as a filter wheel with a plurality of interferial filters 13 is mounted on an axis 14 of a stepper motor 15. On the optical axis A of the elongated body 2 is mounted in the rear part 5 a digital camera 17 with receiving optics 18. An electrical cable 19 is connected to the rear part 5. At the distal end 4 a rotating head 20 with transmitting optics 21 is mounted on the elongated body 2. The elongated body 2 has an inner empty opaque black tunnel 23 for guiding the transmitting light 24 and the receiving light 25.

The receiving optics 18 and the optical path are designed to focus the picture on the wave adjusting means or filter wheel 12, in order to reduce its size and to focus the picture on the digital camera 17 which may be a CCD or CMOS sensor. The black tunnel 23 includes a volume perfectly straight with a cross-section large enough for the transmission of the image. The optical paths of the transmitting light 24 and the receiving light 25 are not perfectly parallel, because the collimation can not be perfect but leads to an optical opening with an angle of about 4°, i.e. the optical paths are slightly conical and not perfect cylindrical. In order to correct the collimation of the light path several collimator lenses (not depicted) may be provided. The light source unit 8 may be an array of different color-producing light sources, especially LEDs, with printed electronic circuit boards which have a metal heat sink, e.g. of aluminum. The heat sink can reach the external surface of the handpiece. The rotating head 20 is provided with an encoder, whereas the encoded rotation is used by the software to adapt the image accordingly. The shape and the amount of space of the elongated body 2 are similar to a standard thin oral endocamera.

In Figures 2A, 2B and 2C a disposable sheath 27 with a disposable cap 28 are shown, which is covering the elongated body 2 in Fig. 1. The disposable cap 28 is mounted in a fixed geometrical position with respect to the disposable cover 27 in order to lie flat on the target tissue 30. For example, in Fig. 2A the disposable cap 28 is provided at an angle of 0° to the optical axis, in Fig. 2B at an angle of 90° to the optical axis and in Fig. 2C at an angle of 25° to the optical axis.

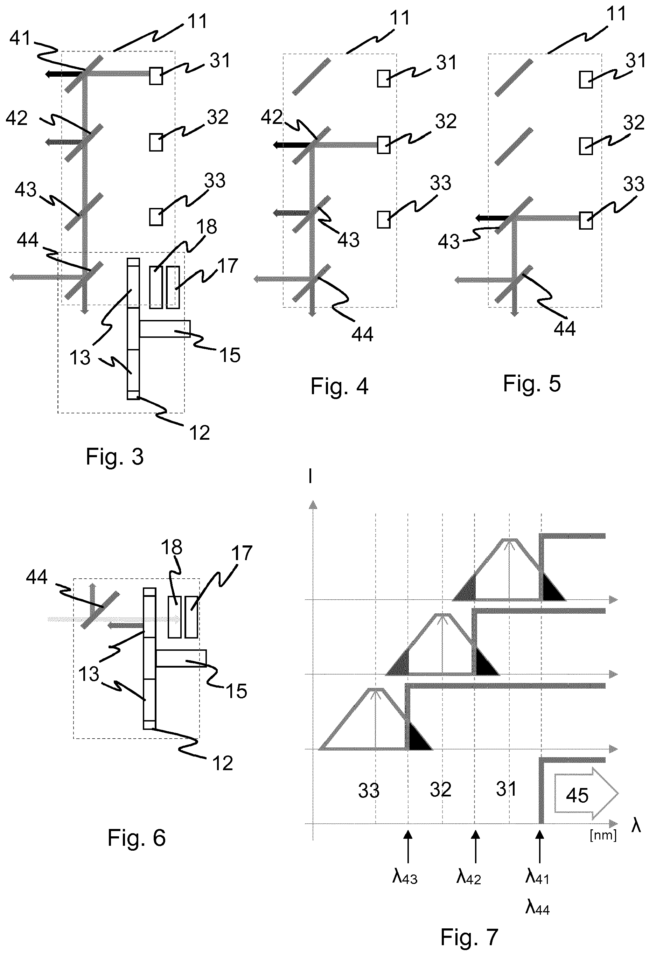

In Fig. 3 the optical beam splitter 11 is shown in more detail. The light source unit 8 covers here three different LED 31, 32 and 33. LED 31 has a blue light spectrum of about 445 nm. LED 32 has a violet light spectrum between 405 and 415 nm and LED 33 has an ultraviolet light spectrum between 385 and 395 nm. The optical splitter 11 is based on interferential filters 41 to 44. In Fig. 3 the blue light of LED 31 is partially reflected at 90° and unwanted wavelengths are removed by the filter 41. In filter 42 and 43 the wanted wavelengths are transmitted and the unwanted wavelengths are reflected at 90°. In filter 44 any residual unwanted wavelengths are transmitted and the wanted wavelengths are reflected at 90°. In this manner the blue light from LED 31 is filtered by the optical beam splitter 11 and transmitted through the elonganted body 2 (Fig. 1). In Figures 4 and 5 a similar filtering process is depicted for LED 32 with violet light and for LED 33 with ultraviolet light.

There may also be provided a white light source (not depicted) covering the solar spectrum of visible light, which is used for a normal picture of the target tissue to be investigated.

As can be seen in Fig. 6 the auto-fluorescence light, which is received from the target tissue, is filtered in filter 44 and the interferial filter 13 of the filter wheel rejects noise (unwanted wavelengths) and transmits only the wanted wavelengths to the digital camera or CCD or CMOS sensor 17.

In Fig. 7 the transmission spectra of the transmitted light from LED 31, LED 32 and LED 33 respectively are shown. At the vertical axis the intensity I is depicted, at the horizontal axis the wavelength λ. The left spectrum is related to LED 33 with a cut-off wavelength λ₄₃ of filter 43. The middle spectrum is related to LED 32 with left and right cut-off wavelengths λ₄₃ and λ₄₂ and the right spectrum is related to LED 31 with left and right cut-off wavelengths λ₄₂ and λ₄₁. The cut-off wavelength λ₄₄ of filter 44 is identical to the cut-off wavelength λ₄₁ of filter 41. The wavelength spectrum 45 of the auto-fluorescence light reflected by the target tissue 30 must be higher than the cut-off wavelength λ₄₄ of filter 44, so that the auto-fluorescence light is transmitted through filter 44.

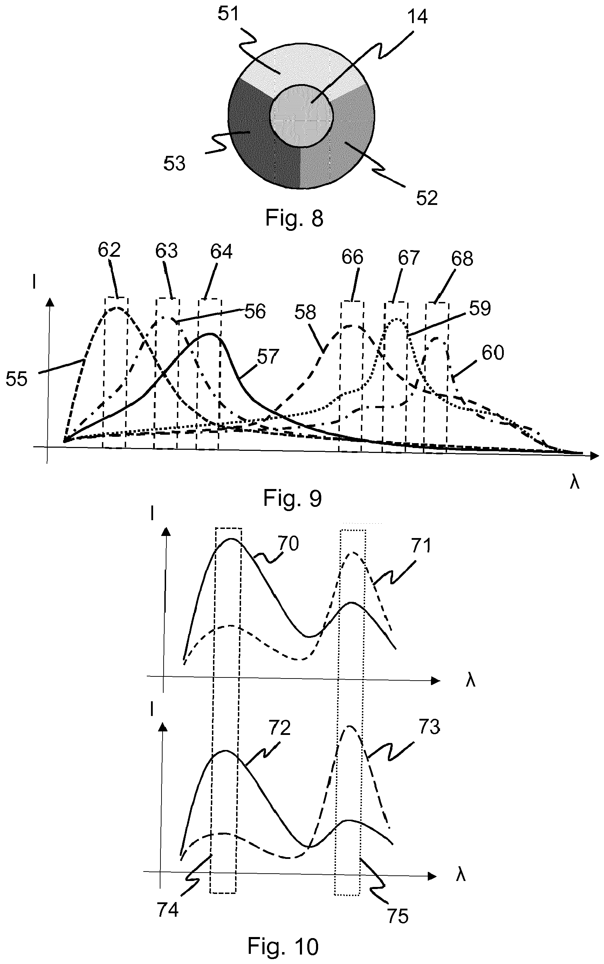

In Fig. 8 the filter wheel 12 is depicted in front view with the axis 14 of stepper motor 15:The receiving filters may be optimized for the target emission corresponding to specific excitation light of the light source 8. In this example a receiving filter 51 is related to LED 31, a receiving filter 52 is related to LED 32 and a receiving filter 53 is related to LED 33. Dependent from the light source which is turned on the receiving filter 51, 52 or 53 is presented in front of the digital camera or CCD or CMOS sensor 17.

Fig. 9 shows the excitation spectrum 55 of LED 31, which is related to the emission spectrum 58 of a first fluorophore A. Transmitting filter band 62 is related to the excitation spectrum 55 and receiving filter band 66 to the emission spectrum 58. Further, excitation spectrum 56 of LED 32 with transmitting filter band 63 is related to emission spectrum 59 with receiving filter band 67 for fluorophore B. At last, excitation spectrum 57 with transmitting filter band 64 for LED 33 is related to emission spectrum 60 with receiving filter band 68 for fluorophore C.

The intraoral inspection apparatus 1 can now be used in two different modes, namely in a single-fluorophore vision mode and in a multi-fluorophores vision mode. In the single-fluorophore vision mode the user selects the fluorophore to be detected. The stepper motor 15 keeps the filter wheel blocked for the specific filter. Hence the apparatus 1 illuminates and receives only signals with respect to that specific fluorophore.

In the multi-fluorophores vision mode in a fast automatic time sequence, apparatus 1 illuminates and receives signals regarding each fluorophore at once. Three frames are acquired for each complete rotation of the filter wheel 12, for example for porphyrin, elastin and collagen. The three frames can be shown individually or combined to obtain a single picture, for example by using three different colors. A subset of fluorophores can be selected by using a graphical user interface (GUI).

In Figure 10 the basic principles of the diagnosing method according to the invention are shown. In the upper part of Fig. 10 the graph of the autofluorescence (AF) spectrum 70 of a healthy tissue and the AF spectrum 71 of a pathological tissue is depicted. In the lower part of Fig. 10 the AF spectrum 72 for a specific tissue at a first excitation wavelength and the AF spectrum 73 for a second excitation wavelength different from the first one is depicted. A specific software program will apply algorithms to evaluate the relationship between the variatons in the receiving bands 74 and 75.

Porphyrines and haemoglobin are the most important fluorophores which can be investigated by the described diagnosing method. Porphyrines are produced by bacteria as copro-porphyrines (aerobic) in the mouth and as proto-IX-porphyrins (anaerobic). In healthy blood the proto-porphyrin takes part in the development of haeme, which means that there are no free porphyrins. The haeme absorbs the photons from the transmitted light without releasing them. However, free proto-IX-porhyrin is accumulated in the cells of the cancerous tissue. See e.g. "Fluorescence spectroscopy for endogenous porphyrins in human facial skin", Seo I et al., February 2009, Proc. Of SPIE Vol. 7161 716103-1".

Haemoglobin can be detected by comparison of the results of the violet light spectrum between 400 and 430 nm and the ultraviolet light spectrum below 400 nm, especially at abouit 380 nm.

For instance for the two porphyrin's bands 74 and 75 in Fig. 10, the elaboration will be related to each single band, to the sum of the bands (total porphyrin energy) and to the difference between the two bands (porphyrin discrimination).

## Claims

1. An intraoral inspection apparatus (1) for diagnosing the physiological and pathological status of an in vivo target tissue (30) being derma or bone tissues or oral mucosa comprising:
a light source unit (8) which is provided with at least one color-producing light source capable of inducing fluorescent lights from healthy and/or diseased sites by irradiating the target tissue;
a wavelength adjusting device (12) which is provided with filter means that transmits, among the fluorescent lights, a light having a wavelength of a fluorescent light from a predetermined diseased site and blocks or attenuates a light having a wavelength other than this wavelength; and
an imaging device (17) which receives the fluorescent light transmitted through the filter means, and converts them into electrical signals,
**characterized in that**
the apparatus (1) is a hand-held device comprising an elongated body (2) including a proximal end (3) and a distal end (4), the body (2) being configured to position the distal end (4) proximate to the target tissue (30), whereas the light source unit (8) is provided at the proximate end (3), and an optical beam splitter (11) with interferential filters (13) is provided in front of the light source unit (8) to remove unwanted wavelengths and to collimate the light beam into the elongated body (2).

2. Intraoral inspection apparatus according to claim 1, **characterized in that** the light source unit (8) is an array of LEDs.

3. Intraoral inspection apparatus according to claim 1 or 2, **characterized in that** the imaging device (17) is a CCD or CMOS sensor.

4. Intraoral inspection apparatus according to one of claims 1 to 3, **characterized in that** the wavelength adjusting device is a filter wheel (12) with a plurality of filter sections (13) each having an individual bandpass characteristic.

5. Intraoral inspection apparatus according to claim 4, **characterized in that** the wheel (12) is driven by a stepper motor (15).

6. Intraoral inspection apparatus according to one of claims 1 to 5, **characterized in that** a stroboscopic means is related to the light source unit (8) for stroboscopic excitation of the illumination light, and an acquisition means related to the imaging device (17) is synchronising the electrical signals obtained by the imaging device (17) from the fluorescent light with the stroboscopic excitation of the illumination light.

7. Intraoral inspection apparatus according to one of claims 1 to 6, **characterized in that** the first and second optical fibers are covered by a disposable sheath (27).

8. Intraoral inspection apparatus according to claim 7, **characterized in that** the disposable sheath (27) comprises a disposable cap (28).

9. Intraoral inspection apparatus according to claim 8, **characterized in that** the disposable cap (28) is fixed at a predetermined angle to the optical axis.
